# EUROPEAN PATENT APPLICATION

(11) **EP 1 314 439 A1**
(43) Date of publication of application: **28.05.2003**
(21) Application number: 02025997.4
(22) Date of filing: 21.11.2002
(51) Int. Cl.: A61L 2/20, A61L 2/18, A61L 2/04, F26B 5/06

(54) **Process for the sterilisation of a powder substance**

(30) Priority: 26.11.2001 IT MI20012488
(71) Applicant: I.C.I. International Chemical Industry Spa, 81030 Cellole (CE) (IT)
(72) Inventor: Scappaticci, Giuseppe, 81030 Cellole (CE) (IT)
(74) Representative: Mancini, Vincenzo, Dr.

(57) **Abstract**

A process for the sterilisation of a powder substance is described; in particular, the invention regards the process for the sterilisation of pharmaceutical active principles in powder, including the use of a lyostat in the presence of Hydrogen Peroxide. The invention process makes it possible to sterilise the active principles mentioned without obtaining toxic substances or a change in colour, and without bringing about a decrease in the stability or in the assay of the active principles.

## Description

The invention refers to a process for the sterilisation of a powder substance.

In particular, the invention regards a process for the sterilisation of pharmaceutical active principles in powder, which involves the use of a lyostat in the presence of Hydrogen Peroxide.

The sterile injectable pharmaceutical products used today are sterilised with diverse techniques such as filtration and subsequent lyophilisation and crystallisation, the sterilisation in the final packaging, using heat, irradiation and treatment with gas.

Irradiation with ultraviolet rays (UV), notwithstanding the high bacterial killing power, is inadequate for the sterilisation of pharmaceutical active principles in powder because it is substantially effective only on the surface, due to the short wavelengths. This peculiarity would obviously make it necessary to lay the powders out finely on very large surfaces.

Among the above mentioned techniques, only the sterilisation with gaseous ethylene oxide and irradiation with β or γ rays are applicable for the sterilisation of pharmaceutical active principles in powder, at the condition that they are already free from foreign particles and have an endotoxine content within the limits indicated by the pharmacopoeia.

Sterilisation with ionising radiations β or γ has been in use for some time; it is used for materials (recipients, glassware, medical instruments etc.) that cannot be sterilised with other methods. Furthermore, for the manufacture and packaging of instruments, it is best to only use materials that can tolerate the quantity of ionising radiations established for the sterilisation. For example, normal glass and some plastic materials become coloured with the radiations; in particular some polymers, due to their constitution and the quantity of ionising radiations absorbed, may undergo both a chemical degradation and a crystalline alteration that changes their physical characteristics,

In the case of powdered substances, it is necessary to verify first of all the compatibility of the irradiation in the quantities (usually ≥5 Kgy, partially toxic for some substances) needed to obtain a bacteria killing effect; the formation of highly reactive free radicals is also important. These bring about the degradation of the active principle and, consequently, a decrease in the assay and alterations in the colour.

Furthermore, as already observed, the irradiation could bring about alterations in the structure of the materials and as a result prevent the application of the technique in question on powders already packaged.

The addition of solid or liquid antiseptics to the pharmaceutical products has its limitations too: these cannot be added to injectable pharmaceutical preparations but only to oral, topic preparations etc., in specific concentrations that, on observation, only have bacteriastatic effects.

On the other hand, gaseous antiseptics such as Ethylene Oxide, Formaldheyde and Ozone are used for the sterilisation of objects and some pharmaceuticals in powder at specific temperature, duration, humidity and concentration conditions.

An ideal sterilising gas must have the following qualities: a strong and rapid activity against bacteria, spores, viruses and mould, possibly at atmosphere pressure; complete inertia towards the product to be treated and the packaging material; an excellent diffusion co-efficient that guarantees easy penetration and total elimination after sterilisation; complete innocuousness for humans and animals; minimum inflammability; easy storage and manipulation; activity without humidity; a reasonable cost and easily sourceable.

None of the three gases presently used have all of the qualities mentioned: Ethylene oxide is the gas that has the most. Formaldheyde does not spread well and high levels of humidity are needed. Ozone does not have a good enough effect on certain germs.

Formaldheyde is very active in a humid environment but as it is very reactive it is only utilised for the sterilisation of objects and environments.

Ozone also needs a high level of humidity to be fully active. It is used preferentially for the sterilisation of surgical instruments and bandages. Both ozonised air, and a cylinder containing a concentrated solution of ozone in Chloro-fluorinated Hydrocarbons are used. However, it is not used very much due to its strong oxidant action, that brings about the alteration of some materials. It is also very toxic, therefore it is necessary to adopt extremely strict precautions in employment.

Ethylene oxide is used for medical-surgical instruments that cannot be sterilised in autoclave (PVC, polyethylene, certain rubbers, etc.). Compared with ionising radiations, it has the advantage of being able to be used for items already packaged, but without causing alterations in the structure of the materials used for the packaging.

Nevertheless, sterilisation with Ethylene oxide is only allowed on materials that cannot be sterilised by heat and is applicable only to certain powdered products using very strict precautions.

The main advantage of sterilisation with Ethylene Oxide, with respect to the sterilisation with β or γ rays, is that it can be used in pharmaceutical plants and hospitals. However, the utilisation of this gas is not completely danger free, this is the main reason why the sterilisation using Ethylene oxide must be carried out by authorised, trained and expert personnel.

To reduce the risks of explosion deriving from the use of pure Ethylene oxide, it is commonly used in a mixture with carbonic anhydride and with Chloro-fluorinated hydrocarbons.

The problem with the Ethylene oxide-Carbonic Anhydride mixture is the great difference of vapour tension between the two gases: there is the risk that Carbonic Anhydride becomes gaseous, leaving a mixture containing substantially Ethylene Oxide, which as a result progressively recuperates its explosive properties. In addition, separation of the two gases may take place during the cooling: after the condensation of Ethylene Oxide only inactive, gaseous Carbonic Anhydride remains.

This problem has nevertheless been overcome using particular mixtures of Ethylene oxide and fluoroalkanes but the de-absorption at room temperature can take even more than 15 hours.

In some cases it is better to resort to the joint action of heat and vacuum to completely eliminate the residue of Ethylene oxide.

Apart from the danger of explosions, Ethylene oxide presents other serious drawbacks: it is a cancerous agent and even small quantities of Ethylene oxide in the material destined to enter into contact with tissue could be particularly dangerous due to the notable emolithic modifications these can bring about; Furthermore, it is extremely difficult to separate the residual gas from the product and, in the presence of water, glycol ethylene forms, the elimination of which is practically impossible; the maximum concentration of Ethylene oxide allowed in the air is 1x10⁻⁴ for a prolonged contact. Handling the material immediately after sterilisation can cause dermatitis. In addition, with some components of the items treated it can also form toxic products: in particular, Chlorine Ion with Ethylene oxide forms Ethylene Chlorohydrin.

It is obvious therefore that both sterilisation with Ethylene Oxide and irradiation with β or γ rays has its drawbacks, basically connected to the residuals of gas in the powder, the formation of undesired substances (Ethylene Glycole, the formation of free radicals), the alteration of the colour of the active principles treated and the formation of degradation products with a consequent decline in the stability and a decrease in the assay.

Other techniques used for the production of sterile substances in powder are the lyophilisation and the crystallisation after opportune sterile solubilisations and filtrations. Even though such techniques guarantee the sterility of the substances treated, they have high costs, due basically to the complexity of the plants. Moreover, in the case of crystallisation, the use of organic solvents and the inevitable decrease in the yield are obviously further drawbacks.

Another well known germicide is Hydrogen Peroxide, generally used in a water solution at a concentration of 3%, for the disinfection of external cuts. Due to the absence of toxic effects it is also used in the foodstuffs sector, in the past it was also used for the sterilisation of milk and water.

In fact, the mechanism of action is due to the development of the oxygen, based on the following reaction:

2 H₂O₂ 2 H₂O + O₂

In the presence of the Catalasi enzyme contained in the cells, the equilibrium of reaction moves to the right producing the development of oxygen.

The combination of H₂O₂ and UV rays is well known for its sporicide and fungicide activity, or else for the sterilisation of materials used to wrap or contain alimentary products.

Even though Hydrogen Peroxide has been used as a disinfectant for more than a century, only in recent years (see, for example, N.A. Klapes et al., Appl. Environ. Microbiol., vol. 56, n.2, p.503-506, 1990) the so called "Hydrogen Peroxide in vapour phase"(VPHP) technique has been developed. This technique makes it possible to use it for the sterilisation of machinery and plants for the pharmaceutical industry. Potentially it can also be used for foodstuffs, pharmaceutical substances, packaging material and fermentators in alternative to sterilisation processes (e.g. overheated vapour, Ethylene Oxide, Formaldheyde) which can have drawbacks such as the degradation of the substances treated, high costs of the plants and lengthy sterilisation operations, development of toxic and cancerous gases.

The US 4169123 describes a method for the sterilisation of medical-surgical instruments and medical instruments in general, including packaged ones, using gaseous Hydrogen Peroxide in the vapour phase at temperatures below 80°C, temperatures presently considered insufficient for an adequate sporicide activity. This method is based on the observation of the sterilising action of Hydrogen Peroxide in vapour phase, based on its ability to penetrate in the porosity of the materials of the objects treated. Furthermore, it can also be observed that the technique is inadequate for the sterilisation of substances, due to the degradation caused.

In fact, notwithstanding the suggestion of Klapes et. al (ibid.) to develop the VPHP methodology applying it to the sterilisation of pharmaceutical products too, as of today no techniques elaborated for this purpose are known of; the reasons for which it has not been applied are substantially ascribable to the notable degradation of the products treated with this method. This makes the VPHP technique substantially incompatible with the sterilisation of substances as discussed, for example, in the US 4169123.

Keeping in mind the drawbacks deriving from the application of the sterilisation techniques known, even today the sterilisation of pharmaceutical active principles in powder, carried out so as to be free of undesired substances (toxic substances, free radicals, degradation products, etc.), alterations in the colour and decrease in the assay, continues to be a problem.

For one object of the invention, a process has now been found for the sterilisation of pharmaceutical active principles in powder, which comprises carrying out the following steps in a lyostat, including a lyophilisation/sterilisation chamber (A) and a condensation chamber (C):
(a) Cooling of the active principle in powder at a temperature between - 60°C and -30°C for 1-8 hours, in the presence of an aqueous solution of Hydrogen Peroxide at a concentration ranging from 25% to 50%, in a quantity between 250 and 1000 ml/m³ of the lyophilisation/sterilisation chamber;
(b) reduction of the pressure from 100 to 300 µHg;
(c) Isolation of the lyophilisation/sterilisation chamber from the condensation chamber.
(d) Heating of the active principle to a temperature between +30°c to +60°C, keeping the active principle at such temperature for 8-24 hours;
(e) Cooling to room temperature.
(f) Recovery of the active principle.

As commonly known, a lyostat is a generator under vacuum of Hydrogen Peroxide at the vaporous state. It assists the movement of the equilibrium of the disassociation reaction of Hydrogen Peroxide towards the formation of water and oxygen and, as noted here, basically includes a lyophilisation/sterilisation chamber in which the product is placed, cooled and heated, a condensation chamber , a depressurisation plant, a cooling plant, a heating plant and a control panel; the operating conditions of the equipment can be regulated and can vary within ample intervals.

Preferably, during the invention process, step (a) is carried out at a temperature of -40°C for 3-6 hours, in the presence of Hydrogen Peroxide at a concentration of 35% in quantity between 250 and 750 ml/m³ of the lyophilisation/sterilisation chamber for active principles in powder having a number of colonies forming units (c.f.u.) /g less or equal to 50; or
from 500 to 1000 ml/m³ of lyophilisation/sterilisation chamber for active principles in powder having more than 50 c.f.u./g;
and the step (b) is carried out at a pressure equal to 150 µHg at a temperature between +40°C and +50°C, maintaining the product a such temperature for 12 hours.

For another object, the invention concerns the use of a lyostat, including a lyophilisation/sterilisation chamber (A), in which the active principle can be placed, and a condensation chamber (C), for the sterilisation of pharmaceutical active principles in powder.

The invention process makes it possible to sterilise pharmaceutical active principles in powder without obtaining toxic substances, free radicals, degradation products or alterations in the colour, or a long term decrease in the stability and assay. Furthermore, the pharmaceutical active principles sterilised according to the invention process, such as Penicillin G clemizole, Ampicillin trihydrate, Ampicillin Benzathine, Amoxycillin Trihydrate, Oxacillin Benzathine, Cloxacillin Benzathine, Cloxacillin sodium, Dicloxacillin Benzathine, Dicloxacillin sodium, Colistine sulphate, Chloramphenicol base, etc., contain a quantity of foreign particles and an endotoxine content within the limits indicated in the pharmacopoeias (e.g. EU III ed., USP XXIV ed., BP 2000); the sterilisation obtained with the invention demonstrates in fact to be able to exercise a quick and efficient activity against bacteria, spores, viruses and moulds, without interfering with the product, and a total innocuousness for humans and animals.

The sterilising action of the invention process is believed to be substantially ascribable to the presence of Hydrogen which is freed from the decomposition of Hydrogen Peroxide, that is thought to interact with the cellular walls of the bacterial cells or fungus, inactivating them.

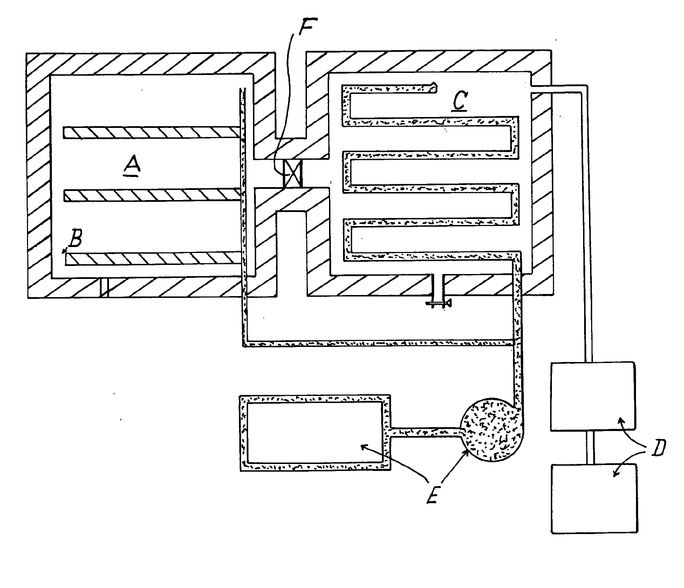

The following description of an example of performance of the invention process refers to the figure that is a schematic representation of the parts of a lyostat mentioned in this description.

According to an example of a preferential performance of the invention process, the active principle in powder, kept in suitable containers, is placed in the lyophilisation/sterilisation chamber(A) in which there are some support pillars (B), in the presence of a solution of Hydrogen Peroxide at a concentration of 35%, in a quantity between 250 and 1000 ml/m³ of lyophilisation/sterilisation chamber, according to the number of microorganism colonies previously determined (according to the specifications mentioned in the USP XXIV ed.) and measured in c.f.u./g.

It is then cooled to -40°C using the production and hot/cold distribution group (E), by circulating a cooling fluid in the pillars, Freon 22 for example. It is kept at that temperature for 2-6 hours. After having lowered the pressure to 150 µHg through group (D) to generate the vacuum, and having isolated the chambers by closing the communication valve (F) between the lyophilisation/sterilisation chamber (A) and the condensation chamber (C) the temperature is brought to +50°C and maintained at such level for 12 hours. Finally, after having cooled to +25°C and recovered the active principle, the powder is filled into glass vials in asepsis conditions.

The following examples illustrate the invention without limiting it.

### EXAMPLE No. 1

100g of Cloxacillin Benzathine has been spread to a thickness of 0,8 cm in an Stainless Steel bowl. A strip with 10 spores of *Bacillus substillis* and 10 spores of Bacillus steathermofilus has been added to the powder.

12 ml of H₂O₂ at a concentration of 35% w/v has been poured into another Stainless Steel bowl subsequently placed in the lyophilisation/sterilisation chamber (0,07 m³) of a lyostat 1002 (5000 ampoules, 2 plates) produced by Criofarma, together with the bowl containing the Cloxacillin Benzathine.

The temperature has been lowered to -40°C and kept at such level for 120 minutes; after having created the vacuum in the lyophilisation/sterilisation chamber up to 150 µHg and having closed the connection valve between the lyophilisation/sterilisation chamber and the condensation chamber, the temperature has been brought to +50°C and kept at such level for 12 hours.

After having cooled to +25°C and unloaded the active principle, the powder is filled in glass vials, in asepsis conditions.

### CONTROLS

### Chemical/physical analysis

Analyses have been carried out on the active principle before and after the invention process has been carried out. The chemical/physical parameters have remain practically unchanged.

### Microbiological controls

The spores of *B. substillis* and of *B. steathermofilus* have been incubated at 55°C and 35°C for seven days. Concurrently a positive control has been carried out. On the positive control growth took place after 12 hours. After seven days no growth had taken place on the spores treated together with the Cloxacillin Benzathine. At the same time a sterility test was carried out on the Cloxacillin Benzathine powder (according to the specifications mentioned in the USP, XXIV ed.), which resulted sterile after fourteen days of incubation.

The following table illustrates the results obtained.

| | Before treatment | After treatment | Limits |
|---|---|---|---|
| Description | White powder | White powder | White, or nearly white powder |
| K.F. | 3,12% | 3,20% | <5% |
| pH | 5,85 | 5,85 | 3,0-6,5 |
| Cloxacillin* | 76,99% | 76,80% | ≥72% |
| Benzathine * | 20,89% | 20,90% | 20-22% |
| Cloxacillin Benzathine* | 98,20% | 98,20% | ≥92% |
| Degradation | 0,67% | 0,69% | ≤5% |
| c.f.u./g | 10,00 | 0,00 | Sterile (0,00) |

| | | | |
|---|---|---|---|
| *- *assay calculated on dry basis* | | | |

It can therefore be observed that the product subjected to the invention process is sterile and, as no degradation has taken place, there have been no modifications to its chemical/physical characteristics.

### EXAMPLE No. 2

100g of Ampicillin Trihydrate has been spread to a thickness of 0,8 cm in a Stainless Steel bowl. Two strips with 10⁶ spores of *Bacillus substillis* and 10⁵ spores of *Bacillus steathermofilus* have been added. 7 ml of H₂O₂ at a concentration of 35% w/v has been poured into another Stainless Steel bowl subsequently placed in the lyophilisation/sterilisation chamber (0,07 m³) of a lyostat 1002 (5000 ampoules, 2 plates) produced by Criofarma, together with a bowl containing Ampicillin Trihydrate.

The temperature has been lowered to -40°C and kept at such level for 120 minutes; after having created vacuum in the lyophilisation/sterilisation chamber up to 150 µHg and having closed the connection valve between the chambers, the temperature has been brought to +50°C and maintained at such level for 6 hours.

After having cooled to +25°C and unloaded the active principle, the powder is filled in glass vials, operating in asepsis conditions.

### CONTROLS

### Chemical-physical analysis

Analyses have been carried out on the active principle before and after the invention process has been carried out. The chemical/physical parameters have remain practically unchanged.

### Microbiological checks

The *B. substillis* and *B. steathermofilus* spores have been incubated at 55°C and 35°C for seven days. A positive check has been carried out simultaneously. On the positive check a growth took place after 12 hours.

After seven days no growth had taken place on the spores treated together with the Ampicillin trihydrate. Concurrently the sterility test has been carried out (according to the specifications indicated in the USP XXIV edition) on the powder of Ampicillin trihydrate, which resulted sterile after fourteen days of incubation.

The following table illustrates the results obtained:

| | Before treatment | After treatment | Limits |
|---|---|---|---|
| Description | White powder | White powder | White powder |
| K.F. | 13,46% | 13,50% | 12.5-15,0% |
| pH | 4,66 | 4,60 | 3,5-5,5 |
| Assay* | 96,77% | 96,50% | 96,0-100,5 |
| Degradation | 0,66% | 0,67% | ≤0,8% |
| c.f.u./g. | 8,00 | 0,00 | Sterile (0,00) |

| | | | |
|---|---|---|---|
| **Assay calculated on dry basis* | | | |

It can therefore be observed that the product subjected to the invention process is sterile and due to the fact that no degradation has taken place, its chemical/physical characteristics have not undergone any modifications.

### EXAMPLE 3

100g of Amoxicillin Trihydrate has been spread to a thickness of 0,8 cm in a Stainless steel bowl and two strips with 10 spores of *Bacillus substillis* and 10 spores of *Bacillus steathermofllus* have been added.

4 ml of H₂O₂ at a concentration of 35% w/v has been poured into another stainless steel bowl, subsequently placed in the lyophilisation/sterilisation chamber (0,07 m³) of a 1002 lyostat (5000 ampoules, 2 plates) produced by Criofarma, together with the bowl containing Amoxycillin trihydrate.

The temperature has been lowered to -40°C and maintained at such level for 120 minutes; after having created a vacuum in the lyophilisation/sterilisation chamber up to 150 µHg and having closed the connection valve between the chambers, the temperature has been increased to +40°C and maintained at such level for 3 hours.

After having cooled to +25°C and unloaded the active principle, the powder is filled into glass vials, operating in asepsis conditions.

### CONTROLS

### Chemical/Physical analysis

Analyses have been carried out on the active principle before and after having subjected the product to the invention process. The chemical/physical parameters have remained almost unchanged.

### Microbiological checks

The spores of *B. substillis* and of *B. steathermofilus* have been incubated at 55°C and 35°C for seven days. Concurrently a positive check has been carried out. On the positive check growth has taken place after 12 hours.

After seven days no growth had taken place on the spores treated together with the Amoxicillin trihydrate. The sterility test was carried out at the same time (according to the specifications indicated in the USP XXIV edition) on the powder of Amoxicillin trihydrate, which resulted to be sterile after fourteen days of incubation.

The following table illustrates the results obtained.

| | Before treatment | After treatment | Limits |
|---|---|---|---|
| Description | White powder | White powder | White, or nearly white powder |
| K.F. | 13,50% | 13.60% | 11,5-14,5% |
| pH | 4,58 | 4,60 | 3,5-5,5 |
| Assay* | 98,28% | 98,10% | 95,0-100,5% |
| Degradation | 0,60% | 0,60% | ≤0,8% |
| c.f.u./g. | 8,00 | 0,00 | Sterile (0,00) |

| | | | |
|---|---|---|---|
| **Assay calculated on dry basis.* | | | |

It can therefore be observed that the product subjected to the invention process is sterile and due to the fact that no degradation has taken place, its chemical/physical characteristics have not undergone any modifications.

## Claims

1. Process for the sterilisation of active pharmaceutical principles in powder, which comprises carrying out the following steps in a lyostat, containing a lyophilisation/sterilisation chamber (A) and a condensation chamber (C):
(a) cooling of the active principle in powder at a temperature between - 60°C and -30°C for 1-8 hours, in the presence of an aqueous solution of Hydrogen Peroxide at a concentration ranging from 25% to 50%, in a quantity between 250 and 1000 ml/m³ of the lyophilisation/sterilisation chamber.
(b) reduction of the pressure from 100 to 300 µHg;
(c) isolation of the lyophilisation/sterilisation chamber from the condensation chamber;
(d) heating of the active principle to a temperature between 30°C and 60°C, keeping the active principle at such temperature for 8-24 hours;
(e) cooling to room temperature;
(f) recovery of the active principle.

2. Process for the sterilisation of pharmaceutical active principles in powder according to Claim 1, in which step (a) is carried out at a temperature equivalent to -40°C for 3-6 hours, in the presence of Hydrogen Peroxide at a concentration of 35% in a quantity between 250 and 750 ml/m³ of the lyophilisation/sterilisation chamber for active principles in powder having a number lower than, or equal to 50 c.f.u./g; or
between 500 and 1000 ml/m³ of the lyophilisation/sterilisation chamber for active principles in powder having a number higher than 50 c.f.u./g; and
step (b) is carried out at a pressure equivalent to 150 µHg at a temperature between 40°C and 50°C, maintaining the product a such a temperature for 12 hours.

3. Process for the sterilisation of pharmaceutical active principles in powder according to claims 1 or 2, in which the active principle is selected from Penicillin G Clemizole, Ampicillin Trihydrate, Ampicillin Benzathine, Amoxycillin Trihydrate, Oxacillin Benzathine, Cloxacillin Benzathine, Cloxacillin sodium, Dicloxacillin Benzathine, Dicloxacillin sodium, Colistine Sulphate, Chloramphenicol base.

4. Use of a lyostat, containing a lyophilisation/sterilisation chamber (A), in which to place the active principle and a condensation chamber (C) for the sterilisation of pharmaceutical active principles in powder.
